# EUROPÄISCHE PATENTSCHRIFT

(11) **EP 1 309 660 B1**
(45) Veröffentlichungstag und Bekanntmachung des Hinweises auf die Patenterteilung: **21.02.2007**
(21) Anmeldenummer: 01936420.7
(22) Anmeldetag: 31.05.2001
(51) Int. Cl.: C08L 59/00

(54) **GLEITMITTELHALTIGE POLYOXYMETHYLENFORMMASSE, IHRE VERWENDUNG UND DARAUS HERGESTELLTER FORMKÖRPER**
POLYOXYMETHYLENE MOULDING COMPOUND CONTAINING A LUBRICANT, THE USE THEREOF AND MOULDED BODIES PRODUCED THEREFROM
MATIERE A MOULER EN POLYOXYMETHYLENE CONTENANT UN LUBRIFIANT, SON UTILISATION ET CORPS MOULE FABRIQUE A PARTIR DE CELLE-CI

(30) Priorität: 15.06.2000 DE 10029533
(43) Veröffentlichungstag der Anmeldung: 14.05.2003
(73) Patentinhaber: Ticona GmbH, 65451 Kelsterbach (DE)
(72) Erfinder: KURZ, Klaus, 65451 Kelsterbach (DE); SCHLEITH, Oskar, 65719 Hofheim (DE)
(86) Internationale Anmeldenummer: PCT/EP2001/006231
(87) Internationale Veröffentlichungsnummer: WO 2001/096470

(56) Entgegenhaltungen:
- WO-A-99/35191
- US-A- 5 416 152
- US-A- 6 046 141

## Beschreibung

Die Erfindung betrifft ein gleitmodifiziertes Polyoxymethylen enthaltend eine Mischung aus einem aliphatischen Ester und einem Polyethylenwachs und seine Verwendung.

Polyoxymethylen, Homo- und Copolymerisate mit einem eingearbeiteten aliphatischen Ester zeichnen sich bei einer tribologischen Paarung mit Kunststoffen oder metallischen Werkstoffen besonders mit Stahl durch sehr geringe Reibungszahlen und geringem Abrieb aus. Die Reibungszahl, z.B. eines mit Esterwachs ausgerüsteten Polyoxymethylen (POM) kann im Vergleich zum jeweiligen unmodifizierten Material um 40% niedriger liegen.
Derartige Polyoxymethylenforrümassen sind im Allgemeinen bekannt, wie zum Beispiel in US 5,559,180 und EP 589354 wo Polyacetal-Formmassen, die u.A. Fettsäureester enthalten können, beschrieben sind. Formmassen, die relative geringe Mengen an Polyethylenwachsen enthalten, sind zum Beispiel aus EP 548692 bekannt, wobei diese Materialien nicht für die Verwendung in tribologischen Anwendungen entwickelt wurden.

Die positive tribologische Eigenschaft der oben erwähnten Mischung POM mit aliphatischem Ester führt allerdings bei der Verarbeitung dieser Produkte zu Problemen. Da die Hersteller von Verarbeitungsmaschinen, wie Extruder und Spritzgießmaschinen für die Aufbereitung der Schmelze ausschließlich Stahlschnecken verwenden, entstehen aufgrund der geringen Reibungszahl in den Einzugszonen dieser Schnecken Förderprobleme. Dies kann aufgrund der geringen Reibung zwischen Granulat und Stahlschnecke zu sehr langen Dosierzeiten und damit zu unwirtschaftlichen Zykluszeiten beim Herstellen der Formteile führen. Im ungünstigsten Fall wird das Material überhaupt nicht in die Schnecke eingezogen.

Abhilfe schafft man dann in der Regel durch Auftrommeln von geringen Mengen Metallsalzen einer Carbonsäure. Dies ist aber in der Praxis ein sehr arbeitsaufwendiger Vorgang.
Eine Einarbeitung dieser Metallsalze in das Produkt ist ebenfalls möglich. Doch müssen hier deutlich höhere Konzentrationen als beim Auftrommeln verwendet werden. Sie können im Polymeren zur Minderung der mechanischen Eigenschaften führen. Auch werden oft beim Spritzgießvorgang Ablagerungen im Werkzeug und Verfärbungen am Fertigteil beobachtet.

Aus der US 6,046,141 ist die Verwendung von oxidierten Polyethylenwachsen bekannt, die als Additive eingesetzt werden um die Abriebfestigkeit von thermoplastischen Formmassen auf Basis von beispielsweise Polyoxymethylen zu verbessern.

Die Aufgabe der vorliegenden Erfindung besteht daher darin, das Einzugsverhalten eines mit einem aliphatischen Ester modifizierten Polyoxymethylens zu verbessern, wobei der Abfall der mechanischen Eigenschaften durch die Zusätze reduziert und die guten tribologischen Eigenschaften beibehalten werden sollen.

Diese Aufgabe wird gelöst durch eine thermoplastische Formmasse enthaltend Komponente (A) 40 bis 99,5 Gew.-Teilen eines Polyoxymethylenhomo- oder Copolymerisats,
Komponente (B) 0,5 bis 10 Gew.-Teile einer Gleitmittelmischung,
Komponente (C) 0 bis 60 Gew.-Teilen Zusatzstoffe,
wobei die Gleitmittelmischung einen aliphatischen Ester und ein Polyethylenwachs enthält und die Summe der Komponenten (A), (B) und (C) stets 100 Gew. Teile ist.. Es wurde überraschend gefunden, daß die Mischung zwischen aliphatischen Estern und Polyethylenwachsen einen Kompromiß zwischen guter Gleitwirkung, geringem Abfall der mechanischen Eigenschaften und einem vertretbaren Einzugsverhalten in die Verarbeitungsmaschinen bewirkt. Die Vorteile der Mischung sind:
- Das Einzugsverhalten in die Verarbeitungsmaschinen wird verbessert
- Die mechanischen Eigenschaften in der Fließnaht des Polyoxmethylens fallen weniger stark ab
- Der Verschleiß an den Fertigteilen verändert sich innerhalb der Meßgenauigkeit von tribologischen Messungen nicht, wenn man die Ergebnisse mit POM, die einen aliphatischen Ester ohne Zusatz von Polyethylenwachs enthalten, vergleicht.
Die Aufgabe konnte somit durch die Verwendung einer Gleitmittelmischung, die neben dem Ester zusätzlich Polyethylenwachs beinhaltet, gelöst werden, wobei ein hochmolekulares, oxidiertes (polares) Polyethylenwachs bevorzugt wird. Obwohl Polyethylenwachse ebenfalls grenzflächenaktive Gleitmittel sind, d.h. auch gute Gleiteigenschaften gegen Stahl haben, besitzen sie nicht die hohe trennende Wirkung wie die aliphatischen Ester, z.B. die Esterwachse.

Als Komponente (A) eignet sich das eingangs genannte Polyoxymethylen Homo-und Copolymerisat. Bei den Polyoxymethylenen (POM), wie sie beispielsweise in der DE-A 29 47 490 beschrieben sind, handelt es sich im Allgemeinen um unverzweigte lineare Polymere, die in der Regel mindestens 80 %, vorzugsweise mindestens 90 %, Oxymethyleneinheiten (-CH₂O-) enthalten. Der Begriff Polyoxymethylene umfaßt dabei sowohl Homopolymere des Formaldehyds oder seiner cyclischen Oligomeren wie Trioxan oder Tetroxan als auch entsprechende Copolymere.

Homopolymere des Formaldehyds oder Trioxans sind solche Polymere, deren Hydroxylendgruppen in bekannter Weise chemisch gegen Abbau stabilisiert sind, z.B. durch Veresterung oder Veretherung. Copolymere sind Polymere aus Formaldehyd oder seinen cyclischen Oligomeren, insbesondere Trioxan, und cyclischen Ethern, cyclischen Acetalen und/oder linearen Polyacetalen. Derartige POM-Homo- oder Copolymerisate sind dem Fachmann an sich bekannt und in der Literatur beschrieben. Ganz allgemein weisen diese Polymere mindestens 50 mol-% an wiederkehrenden Einheiten -CH₂O- in der Polymerhauptkette auf. Die Homopolymeren werden im allgemeinen durch Polymerisation von Formaldehyd oder Trioxan hergestellt, vorzugsweise in der Gegenwart von geeigneten Katalysatoren.
Im Rahmen der Erfindung werden POM-Copolymere als Komponente (A) bevorzugt, insbesondere solche, die neben den wiederkehrenden Einheiten -CH₂O- noch bis zu 50, vorzugsweise von 0,1 bis 20 und insbesondere 0,5 bis 10 mol-% an wiederkehrenden Einheiten 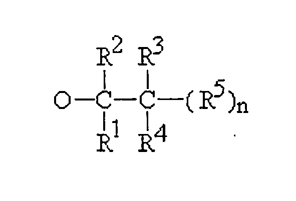 enthalten, wobei R¹ bis R⁴ unabhängig voneinander ein Wasserstoffatom, eine C₁- bis C₄-Alkylgruppe oder eine halogensubstituierte Alkylgruppe mit 1 bis 4 C-Atomen und R⁵ eine -CH₂-, -CH₂O-, eine C₁- bis C₄-Alkyl- oder C₁- bis C₄-Haloalkyl substituierte Methylengruppe oder eine entsprechende Oxymethylengruppe darstellen und n einen Wert im Bereich von 0 bis 3 hat. Vorteilhafterweise können diese Gruppen durch Ringöffnung von cyclischen Ethern in die Copolymere eingeführt werden. Bevorzugte cyclische Ether sind solche der Formel 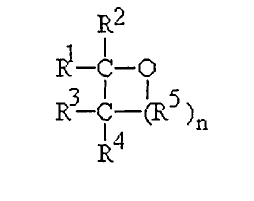 wobei R¹ bis R⁵ und n die obengenannte Bedeutung haben. Nur beispielsweise seien Ethylenoxid, 1,2-Propylenoxid, 1,2-Butylenoxid, 1,3-Butylenoxid, 1,3-Dioxan, 1,3-Dioxolan und 1,3-Dioxepan als cyclische Ether sowie lineare Oligo- oder Polyformale wie Polydioxolan oder Polydioxepan als Comonomere genannt. Besonders vorteilhaft sind Copolymere aus 99,5 - 95 Mol-% Trioxan und 0,5 bis 5 mol-% einer der vorgenannten Comonomere.

Als Komponente (A) ebenfalls geeignet sind Oxymethylenterpolymerisate, die beispielsweise durch Umsetzung von Trioxan, einem der vorstehend beschriebenen cyclischen Ether und mit einem dritten Monomeren, vorzugsweise einer bifunktionellen Verbindung der Formel 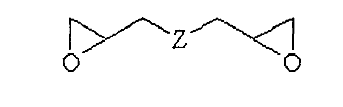 wobei Z eine chemische Bindung, -O- oder -ORO- (R=C₁- bis C₈-Alkylen oder C₂- bis C₈-Cycloalkylen) ist, hergestellt werden.

Bevorzugte Monomere dieser Art sind Ethylendiglycid, Diglycidylether und Diether aus Glycidylen und Formaldehyd, Dioxan oder Trioxan im Molverhältnis 2 :1 sowie Diether aus 2 mol Glycidylverbindung und 1 mol eines aliphatischen Diols mit 2 bis 8 C-Atomen wie beispielsweise die Diglycidylether von Ethylenglykol, 1,4-Butandiol, 1,3-Butandiol, Cyclobutan-1,3-diol, 1,2-Propandiol und Cyclohexan-1,4-diol, um nur einige Beispiele zu nennen.

Verfahren zur Herstellung der vorstehend beschriebenen POM-Homo- und Copolymerisate sind dem Fachmann bekannt und in der Literatur beschrieben.

Die bevorzugten POM-Copolymere haben Schmelzpunkte von mindestens 150 °C und Molekulargewichte (Gewichtsmittelwert) M_{w} im Bereich von 5 000 bis 200 000, vorzugsweise von 7 000 bis 150 000. Endgruppenstabilisierte POM-Polymerisate, die an den Kettenenden C-C-Bindungen aufweisen, werden besonders bevorzugt. Die eingesetzten POM-Polymere haben im allgemeinen einen Schmelzindex (MVR-Wert 190/2,16) von 2 bis 50 cm³/10 min (ISO 1133).

Als Gleitmittelmischung (B) enthält die erfindungsgemäße Formasse eine Mischung aus mindestens einem aliphatischen Ester und mindestens einem Polyethylenwachs. Bevorzugt enthält die erfindungsgemäße Formmasse 1 bis 3 Gewichtsteile der Gleitmittelmischung (B). Bevorzugt besteht die Gleitmittelmischung (B) aus einem aliphatischen Ester und einem Polyethylenwachs im Verhältnis von 1 zu 2 Gewichtsteilen. Aliphatische Ester können beispielsweise sein: Ester aus gesättigten oder ungesättigten aliphatischen Carbonsäuren, bevorzugt solche, die 10-100 Kohlenstoffatome enthalten, besonders bevorzugt sind Esterwachse, Stearylstearat, Behenylbehenat, Isostearylstearat, Glyzerinmonostearat, Glyzerinmonoisostearat, Glyzerindistearat, Pentaerythrittetrastearat, Pentaerythrittetrabehenat, Montansäureester, Montansäureester teilverseift. Es können auch Mischungen aus verschiedenen Estern in beliebigen Mischungsverhältnissen eingesetzt werden.
Als Polyethylenwachse können Wachse mit einer Dichte von 0,92 bis 0,98 g/cm³ und mit einem Tropfpunkt von 100 bis 145°C sowie einer Viskositätszahl bei 140°C von 100 bis 100000 mPa s verwendet werden. Auch hier können Mischungen in beliebigem Verhältnis verwendet werden.
Bevorzugt werden als Polyethylenwachse oxidierte Polyethylenwachse verwandt.
Das bevorzugte oxidierte Polyethylenwachs ist ein hochmolekulares, polares Wachs und besitzt im allgemeinen eine Säurezahl von 5 bis 60 mg KOH/g, vorteilhaft von 5 bis 50 mg KOH/g, insbesondere von 5 bis 25 mg KOH/g und eine Viskosität von 3000 bis 100000 mPa•s bei 140°C.
Die Besonderheit des oxidierten Polyethylenwachses sind die mit Sauerstoff oberflächengebundenen, funktionellen Gruppen. Sie werden gezielt durch oxidative Nachbehandlung erzeugt. Durch die oxidative Nachbehandlung des Polyethylenwachses wird die Affinität zum POM verbessert. Im Vergleich zu anderen Polyethylenwachsen fällt die Dehnung in der Fließnaht weniger stark ab und der Abrieb beim Gleitvorgang wird geringer. Dieser zusätzliche Vorteil ist auch aus EP 0 905 190 bekannt. Gleitmittel, die zur Verwendung in der erfindungsgemäßen Gleitmittelmischung geeignet sind, werden auch beschrieben in Gächter/Müller, "Taschenbuch der Kunststoff-Additive", 3. Ausgabe, Carl Hanser Verlag München/Wien 1994, Seite 478-504 worauf Bezug genommen wird.

Zusatzstoffe (C) können sein Verarbeitungshilfen, Füll -, Verstärkungs und / oder polymere Gleitstoffe; als Beispiel seien zum Beispiel Formaldehydfänger, Säurefänger, Antioxidantien, UV-Stabilisatoren, Haftvermittler, Nukleierungsmittel und Entformungshilfen oder auch Carbonfasern, Aramidfasern, Glasfasern , Glaskugeln, Calciumcarbonat, Wollastonit, Siliciumdioxid und zusätzliche Gleitstoffe sowie deren Mischungen genannt, ohne jedoch den Umfang auf die genannten Beispiele zu beschränken. Zusätzliche Gleitstoffe sind zum Beispiel ultrahochmolekulares Polyethylen (PE-UHMW), Polytetrafluorethylen (PTFE) und Pfropf-Copolymer, welches ein Produkt einer Pfropfreaktion aus einem Olefin-Polymer und einem Acrylnitril/Styrol-Copolymer ist, oder Mischungen davon. Die Komponenten (A) bis (C) ergänzen sich dabei stets zu 100 Gewichtsteilen. Das erfindungsgemäße, gleitmodifizierte Polyoxymethylen zeichnet sich durch eine verbesserte Verarbeitbarkeit aus und die Bindenahtfestigkeit erhöht sich im Vergleich zu einem POM das nur mit aliphatischen Estern in vergleichbarer Konzentration modifiziert ist. Das gute Reib- und Verschleißverhalten bleibt weitgehend erhalten. Das Material eignet sich zur Herstellung von Formteilen - jeglicher Art. Als Gleitpartner zu metallischen Werkstoffen, besonders zu Stahl aber auch zu härteren Kunststoffen, wie Polybutylenterephthalat, sind die tribologischen Eigenschaften des Materials von besonderem Nutzen. Die folgenden Beispiele sollen dem Fachmann die Vorteile der vorliegenden Erfindung verdeutlichen.

### Beispiele

Für das Vergleichsbeispiel A und die erfindungsgemäßen Beispiele 1-4 wurde ein Copolymerisat aus Trioxan und Dioxolan mit einem Volumenschmelzindex MVR 190/2,16 von 8 cm³/10min verwendet. Das Copolymerisat wurde mit den in Tabelle1 aufgeführten Additiven versetzt.

**Tabelle 1.**

| Rezepturbestandteile in Gew.-Teile | Beispiel A Vergleich | Beispiel 1 | Beispiel 2 | Beispiel 3 | Beispiel 4 |
|---|---|---|---|---|---|
| **POM-Copolymer** | **98** | **98** | **98** | **98** | **98** |
| **Esterwachs** | **2** | **1,2** | **1,0** | **0,8** | **0,6** |
| **PE-Wachs oxidiert** | **-** | **0,8** | **1,0** | **1,2** | **1,4** |

Das POM-Copolymerisat mit den jeweiligen Additiven wurde in einem schnellaufenden Fluidmischer, Diosna V 100 (Firma Dierks u. Söhne, Osnabrück, Bundesrepublik Deutschland) gemischt und in einem Doppelschneckenextruder ZE 25 x 33 D (Firma Berstorff, Hannover, Bundesrepublik Deutschland) bei einer Massetemperatur von 200 °C aufgeschmolzen und anschließend granuliert. Das Granulat wurde acht Stunden bei 120 °C getrocknet und anschließend zu Probekörpern für mechanische und tribologische Prüfungen gespritzt. Als Spritzgießmaschine diente der Typ KM 90/210 B (Firma Krauss Maffei, München, Bundesrepublik Deutschland). Die Verarbeitungsbedingungen wurden nach den Empfehlungen der ISO 9988-2, Stoff-Norm für POM, gewählt.

### Messungen:

### Rheologische Eigenschaften

MVR 190/2,16 nach ISO 1133

### Mechanische Eigenschaften

Zugversuch nach ISO 527 Teil 1 und 2

### Verschleißmessungen

Der Abrieb wurde auf einer Verschleißwelle - eine rotierende Welle, auf die zylindrische Probekörper mit 12 mm Durchmesser aus dem zu prüfenden Werkstoff gepreßt werden - gemessen. Das Verschleißvolumen wird in Abhängigkeit von der Zeit bestimmt. Das Testprinzip entspricht nach ISO/DIS 7148-2 dem "pin on ring" Prinzip.

### Prüfbedingungen:

| | |
|---|---|
| Werkstoff Welle | Stahl |
| Wellendurchmesser | 65 mm |
| Rauhtiefe Rz | ca. 0,8 µm |
| Belastung | 3,1 N |
| Gleitgeschwindigkeit | 136 m/min |
| Versuchsdauer | 60 h |

### Ermitteln der Reibungszahl

Die Reibungszahl wurde auf einen Prüfstand mit oszillierender Bewegung durchgeführt. Auf eine sich hin- und her bewegende Platte, hergestellt aus dem zu prüfenden Material, wird eine Kugel mit definierter Normalkraft gepreßt. Die Platte versucht durch die Reibung die Kugel, die an Plattfedern aufgehängte ist, zu verschieben. Die mit induktiven Aufnehmern gemessene Reibungskraft dividiert durch die Normalkraft ergibt die Reibungszahl.
Prüfbedingungen :
Probekörper a) Stahlkugel 12,7 mm ∅
Probekörper b) Platte 20x10x4 mm

Die Meßergebnisse sind in Tabelle 2 aufgeführt.

**Tabelle 2**

| Merkmal | Beispiel A Vergleich | Beispiel 1 | Beispiel 2 | Beispiel 3 | Beispiel 4 |
|---|---|---|---|---|---|
| **MVR 190/2,16 [cm³/10min]** | **8,6** | **8,5** | **8,6** | **8,7** | **8,7** |
| **Dosierzeit an der Spritzgießmaschine [s]** | ***)** | **32** | **31,2** | **28,8** | **27,9** |
| **Bindenahtfestigkeit [MPa]** | **47,5** | **59** | **59** | **60** | **61** |
| **Bruchdehnung an der Bindenaht [%]** | **3,8** | **7,0** | **7,0** | **7,8** | **9,2** |
| **Verschleißvolumen [mm³]** | **0,62** | **0,68** | **0,71** | **0,71** | **0,77** |
| **Reibungszahl** | **0,19** | **0,24** | **0,24** | **0,27** | **0,3** |

| | | | | | |
|---|---|---|---|---|---|
| *) Das Material konnte ohne Vorbehandlung (Auftrommeln von 0,1% Magnesiumstearat) nicht verarbeitet werden. | | | | | |

Die Beispiele zeigen, daß bei den Mischungen Esterwachs mit oxidiertem Polyethylenwachs (Rezepturen 1-4) im Vergleich zu Esterwachs allein (Rezeptur A) das Einzugsverhalten in die Spritzgießmaschine verbessert wird, d. h. die Dosierzeit wird mit steigendem Gehalt an oxidiertem Polyethylenwachs reduziert. Zusätzlich wird eine Zunahme der Bindenahtfestigkeit beobachtet. Das Verschleißvolumen bei den tribologischen Messungen bleibt im Rahmen der Meßgenauigkeit weitgehend gleich.
Aus den Beispielen 1-4 ist aber auch zu entnehmen, daß die Reibungszahl mit zunehmendem Polyethylenwachs zunimmt. Für Anwendungsfälle, bei denen eine möglichst niedrige Reibungszahl benötigen wird, ist daher die Gleitmittelmischung Wachsester/Polyethylenwachs sorgfältig abzustimmen.

## Patentansprüche

1. Thermoplastische Formmasse enthaltend
Komponente (A) 40 bis 99,5 Gew.-Teilen eines Polyoxymethylenhomo- oder Copolymerisats
Komponente (B) 0,5 bis 10 Gew.-Teile einer Gleitmittelmischung
Komponente (C) 0 bis 60 Gew.-Teile Zusatzstoffe,
wobei die Gleitmittelmischung einen aliphatischen Ester einer Carbonsäure mit 10 bis 100 Kohlenstoffatomen und ein Polyethylenwachs enthält und die Summe der Komponenten (A), (B) und (C) stets 100 Gew. Teile ist.

2. Formmasse nach Anspruch 1, wobei die Gleitmittelmischung, Komponente (B), aus einem aliphatischen Ester und einem Polyethylenwachs im Verhältnis von 1 zu 100 bis 100 zu 1 Gew.-Teilen besteht.

3. Formmasse nach Anspruch 1 oder 2, wobei die Gleitmittelmischung, Komponente (B), aus einem aliphatischen Ester und einem Polyethylenwachs in einem Verhältnis von 1 zu 2 Gew.-Teilen besteht.

4. Formmasse nach einem oder mehreren der Ansprüche 1 bis 3, wobei die Formmasse 1 bis 3 Gewichtsteile der Gleitmittelmischung, Komponente (B), enthält.

5. Formmasse nach einem oder mehreren der Ansprüche 1 bis 4, wobei die Gleitmittelmischung aus einem Esterwachs und einem hochmolekularen, Polyethylenwachs mit einer Säurezahl von 5 bis 60 mg KOH/g und einer Viskosität von 3000 bis 100000 mPa*s bei einer Temperatur von 140°C besteht.

6. Formmasse nach einem oder mehreren der Ansprüche 1 bis 5, wobei als Polyethylenwachs ein oxidiertes Polyethylenwachs eingesetzt wird.

7. Formmasse nach einem oder mehreren der Ansprüche 1 bis 6, wobei die Komponente (C) ausgewählt ist aus einem oder mehreren der Füllstoffe Kreide, Talk, Wollastonit, Glimmer, Zinkoxid, Siliciumdioxid, der Verstärkungsstoffe Glasfasern, Aramidfasem, Kohlefasem und/oder organischen Hochmodulfasern und/oder der polymeren Gleitstoffe Polytetrafluorethylen in Pulver- und/oder Faserform, UHMW-Polyethylen, Pfropf-Polymerisate erhalten aus der Pfropfreaktion von Polyethylen, Acrylnitril/Styrol-Copolymerisat (SAN).

8. Verwendung der thermoplastischen Formmasse nach einem oder mehreren der Ansprüche 1 bis 7 zur Herstellung von Formkörpern und Folien.

9. Verwendung der thermoplastischen Formmasse nach einem oder mehreren der Ansprüche 1 bis 7 zur Herstellung eines Formteils, welches als Gleitpartner zu metallischen Werkstoffen oder härteren Kunststoffen eingesetzt wird.

10. Formkörper, hergestellt aus einer thermoplastischen Formmasse nach einem oder mehreren der Ansprüche 1 bis 7.

## Claims

1. A thermoplastic molding composition comprising
component (A) from 40 to 99.5 parts by weight of a polyoxymethylene homo- or copolymer
component (B) from 0.5 to 10 parts by weight of a lubricant mixture
component (C) from 0 to 60 parts by weight of additives,
where the lubricant mixture comprises an aliphatic ester of a carboxylic acid having from 10 to 100 carbon atoms and a polyethylene wax, and the entirety of components (A), (B), and (C) is always 100 parts by weight.

2. The molding composition as claimed in claim 1, where the lubricant mixture, component (B), is composed of an aliphatic ester and of a polyethylene wax in a ratio of 1:100 to 100:1 parts by weight.

3. The molding composition as claimed in claim 1 or 2, where the lubricant mixture, component (B), is composed of an aliphatic ester and of a polyethylene wax in a ratio of 1:2 parts by weight.

4. The molding composition as claimed in one or more of claims 1 to 3, wherein the molding composition comprises from 1 to 3 parts by weight of the lubricant mixture, component (B).

5. The molding composition as claimed in one or more of claims 1 to 4, where the lubricant mixture is composed of an ester wax and of a high-molecular-weight polyethylene wax with an acid value of from 5 to 60 mg KOH/g and with a viscosity of from 3000 to 100,000 mPa*s at a temperature of 140°C.

6. The molding composition as claimed in one or more of claims 1 to 5, where an oxidized polyethylene wax is used as polyethylene wax.

7. The molding composition as claimed in one or more of claims 1 to 6, where component (C) has been selected from one or more of the fillers chalk, talc, wollastonite, mica, zinc oxide, silicon dioxide, the reinforcing materials glass fibers, aramid fibers, carbon fibers, and/or organic high-modulus fibers, and/or the polymeric lubricants polytetrafluoroethylene in powder and/or fiber form, UHMW polyethylene, graft polymers obtained from the graft reaction of polyethylene, acrylonitrile-styrene copolymer (SAN).

8. The use of the thermoplastic molding composition as claimed in one or more of claims 1 to 7 for producing moldings and films.

9. The use of the thermoplastic molding composition as claimed in one or more of claims 1 to 7 for producing a molding which is used as a sliding partner for metallic materials or for relatively hard plastics.

10. A molding produced from a thermoplastic molding composition as claimed in one or more of claims 1 to 7.

## Revendications

1. Mélange à mouler thermoplastique contenant
composant (A) : 40 à 99,5 parties en poids d'un homo- ou copolymère polyoxyméthyléné,
composant (B) : 0,5 à 10 parties en poids d'un mélange lubrifiant,
composant (C) : 0 à 60 parties en poids d'additifs
le mélange lubrifiant contenant un ester aliphatique d'un acide carboxylique ayant 10 à 100 atomes de carbone et une cire de polyéthylène, la somme des composants (A), (B) et (C) étant toujours de 100 parties en poids.

2. Mélange à mouler selon la revendication 1, dans lequel le mélange lubrifiant, le composant (B), est constitué d'un ester aliphatique et d'une cire de polyéthylène selon un rapport de 1:100 à 100:1 parties en poids.

3. Mélange à mouler selon la revendication 1 ou 2, dans lequel le mélange lubrifiant, le composant (B), est constitué d'un ester aliphatique et d'une cire de polyéthylène selon un rapport de 1:2 parties en poids.

4. Mélange à mouler selon l'une ou plusieurs des revendications 1 à 3, dans lequel le mélange à mouler contient 1 à 3 parties en poids du mélange lubrifiant, le composant (B).

5. Mélange à mouler selon l'une ou plusieurs des revendications 1 à 4, dans lequel le mélange lubrifiant est constitué d'une cire d'ester et d'une cire de polyéthylène à grande masse moléculaire, ayant un indice d'acide de 5 à 60 mg KOH/g et une viscosité de 3000 à 100 000 mPa.s à une température de 140°C.

6. Mélange à mouler selon l'une ou plusieurs des revendications 1 à 5, dans lequel on utilise en tant que cire de polyéthylène une cire de polyéthylène oxydée.

7. Mélange à mouler selon l'une ou plusieurs des revendications 1 à 6, dans lequel le composant (C) est choisi parmi une ou plusieurs des matières de charge craie, talc, wollastonite, mica, oxyde de zinc, dioxyde de silicium, les matières de renfort fibres de verre, fibres aramides, fibres de carbone et/ou fibres organiques à grand module d'élasticité, et/ou les lubrifiants polymères que sont le polytétrafluoréthylène sous forme d'une poudre et/ou de fibres, le polyéthylène à ultra-haute masse moléculaire, les polymères greffés obtenus par une réaction de greffage de polyéthylène, le copolymère acrylonitrile/styrène (SAN).

8. Utilisation du mélange à mouler thermoplastique selon l'une ou plusieurs des revendications 1 à 7 pour fabriquer des objets moulés et des feuilles.

9. Utilisation du mélange à mouler thermoplastique selon l'une ou plusieurs des revendications 1 à 7 pour fabriquer un objet moulé qui est utilisé en tant que partenaire de glissement de matériaux métalliques ou de matériaux plastiques plus durs.

10. Objet moulé fabriqué à partir d'un mélange à mouler thermoplastique selon l'une ou plusieurs des revendications 1 à 7.
